(19) Europäisches Patentamt / European Patent Office / Office européen des brevets

(11) **EP 1 801 588 A1**

(12) **EUROPÄISCHE PATENTANMELDUNG**

(43) Veröffentlichungstag:
**27.06.2007 Patentblatt 2007/26**

(51) Int Cl.:
***G01N 33/543*** *(2006.01)*

(21) Anmeldenummer: **05028312.6**

(22) Anmeldetag: **23.12.2005**

(84) Benannte Vertragsstaaten:
**AT BE BG CH CY CZ DE DK EE ES FI FR GB GR HU IE IS IT LI LT LU LV MC NL PL PT RO SE SI SK TR**
Benannte Erstreckungsstaaten:
**AL BA HR MK YU**

(71) Anmelder:
- **Micronas GmbH**
  **79108 Freiburg i. Br. (DE)**
- **Micronas Holding GmbH**
  **79108 Freiburg i.Br. (DE)**

(72) Erfinder:
- **Lehmann, Mirko, Dr. rer. nat.**
  **79117 Freiburg (DE)**
- **Freund, Ingo, Dipl.-Ing. (FH)**
  **79117 Freiburg (DE)**
- **Klapproth, Holger, Dr.**
  **79108 Freiburg (DE)**
- **Mohry, Sonja**
  **79108 Freiburg (DE)**

(74) Vertreter: **Huwer, Andreas**
**Grünwälderstrasse 10-14, Postfach 1305**
**79013 Freiburg i. Br. (DE)**

(54) **Verfahren und Vorrichtung zur Bestimmung der Konzentration von in einer Probe enthaltenen Liganden**

(57) Bei einem Verfahren zur Bestimmung der Konzentration von in einer zu untersuchenden Probe enthaltenen Liganden (6) werden an einer Teststelle auf einem Träger Rezeptoren (5) Immobilisiert, die mit den Liganden (6) eine spezifische Bindung eingehen können. Die Probe wird mit der Teststelle in Kontakt gebracht und es wird mindestens ein die Häufigkeit der Bindungen zwischen den Liganden (6) und den Rezeptoren (5) repräsentierender erster Messwert erfasst Danach wird eine Sättigungslösung, welche die Liganden (ö) In höherer Konzentration enthält als die Probe, derart mit den Rezeptoren (5) in Kontakt gebraucht wird, dass im Wesentlichen alle an der Teststelle befindlichen Rezeptoren (5) an einen Liganden (6) gebunden sind. Danach wird zur Bestimmung der Flächenbelegung der Teststelle mit den Liganden (6) ein zweiter Messwert erfasst Mit Hilfe des ersten und zweiten Messwerts wird die Konzentration der Liganden (ö) in der Probe bestimmt.

Fig. 4

EP 1 801 588 A1

**Beschreibung**

Die Erfindung betrifft ein Verfahren zur Bestimmung der Konzentration von in einer zu untersuchenden Probe enthaltenen Liganden, wobei an einer Teststelle auf einem Träger Rezeptoren immobilisiert werden, die mit den Liganden eine spezifische Bindung eingehen können, wobei die Probe mit der Teststelle in Kontakt gebracht wird, wobei mindestens ein die Häufigkeit der Bindungen zwischen den Liganden und den Rezeptoren repräsentierender erster Messwert erfasst wird. Die Erfindung betrifft ferner ein Verfahren zur Bestimmung der Konzentrationen von In einer zu untersuchenden Probe enthaltenen Liganden, wobei auf einem Träger an mindestens einer Teststelle Rezeptoren immobilisiert werden, die mit den Liganden eine spezifische Bindung eingehen können, wobei die Probe für eine Zeitdauer mit der mindestens einen Teststelle In Kontakt gebracht und danach von dieser entfernt wird. Außerdem betrifft die Erfindung eine Vorrichtung zur Bestimmung der Konzentration von in einer zu untersuchenden Probe enthaltenen Liganden, mit einem Träger, an dessen Oberfläche mindestens eine Teststelle angeordnet ist, an der Rezeptoren immobilisiert sind, die beim Kontaktieren der Liganden mit diesen eine spezifische Bindung eingehen, mit wenigstens einem Detektor zur Erfassung von Messwerten, die von der Häufigkeit der Liganden-Rezeptor-Bindungen abhängig sind.

Aus WO 2004/042399 A1 ist eine Vorrichtung bekannt, bei der an der Oberfläche eines Trägers mehrere seitlich voneinander beabstandete Teststellen vorgesehen sind, an denen jeweils ein Detektor und ein Flächenbelegungssensor in die Trägeroberfläche eingelassen sind. Die einzelnen Detektoren und Flächenbelegungssensoren sind jeweils mit Rezeptoren beschichtet, die für einen in der zu untersuchenden Probe enthaltenen Liganden bindungsspezifisch sind. Mit Hilfe der Flächenbelegungssensoren wird die Flächenbelegung der Trägeroberfläche mit den Rezeptoren ermittelt, bevor die Probe mit den Rezeptoren in Kontakt gebracht wird. Dann wird die Probe auf die Trägeroberfläche aufgebracht und es wird mittels der Detektoren eine Lumineszenzstrahlung empfangen, die in Abhängigkeit von der Bindung des Liganden an den Rezeptor auftritt. Aus den so erhaltenen Messwerten für die Lumineszenzstrahlung und die Flächenbelegung sowie einer bekannten Bindungskonstante wird mit Hilfe des Massenwirkungsgesetzes die Konzentration der Liganden in der Probe berechnet. Durch die Verwendung der Flächenbelegungssensoren kann eine Kalibrierung der Vorrichtung mittels einer Verdünnungsreihe entfallen. Da für jede Teststelle zusätzlich zu dem Detektor ein Flächenbelegungssensor benötigt wird, erfordert das Verfahren eine relativ aufwändige Sensorik, insbesondere wenn eine große Anzahl von Teststellen benötigt wird.

Aus der Praxis ist ferner ein Verfahren zur Bestimmung der Konzentrationen von mindestens zwei in einer zu untersuchenden Probe enthaltenen Liganden unterschiedlicher Ligandenarten bekannt. Dabei weisen Liganden einer ersten Ligandenart eine höhere Konzentration auf als Liganden einer zweiten Ligandenart. Auf einem Träger werden an unterschiedlichen Teststellen Rezeptoren unterschiedlicher Rezeptorarten immobilisiert, Rezeptoren einer ersten Rezeptorart sind für die erste Ligandenart und Rezeptoren einer zweiten Rezeptorart für die zweite Ugandenart bindungsspezifisch. Die Probe wird für eine vorgegebene Zeitdauer mit den Teststellen in Kontakt gebracht und danach von den Teststellen entfernt. Die Zeitdauer wird so gewählt, dass jeweils nur ein Teil der Rezeptoren der einzelnen Rezeptorarten an einen Liganden binden. Anschließend wird für jede Teststelle ein die Häufigkeit der Bindungen zwischen den Liganden und den Rezeptoren repräsentierender zweiter Messwert erfasst. Bei Proben, bei denen die Konzentrationen der Liganden der einzelnen Ligandenarten stark unterschiedlich sind, ermöglicht das Verfahren Jedoch nur eine geringe Messgenauigkeit, da bei der Messwerterfassung an der die Rezeptoren der ersten Rezeptorart aufweisenden Teststelle aufgrund der hohen Konzentration der Liganden der ersten Ligandenart sehr viele Liganden und an der die zweiten Rezeptoren aufweisenden Teststelle nur wenige Liganden an die Rezeptoren gebunden sind. Wegen des nichtlinearen, etwa sigmoidalen Verlaufs der Messkennlinie (Messsignal als Funktion der Konzentration) ergibt sich dann jeweils ein ungünstiger Arbeitspunkt, in dem die Kennlinie nur eine geringe Steigung aufweist.

Es besteht deshalb die Aufgabe, eine Vorrichtung der eingangs genannten Art zu schaffen, die einen einfachen und kostengünstigen Aufbau sowie eine hohe Messauflösung ermöglicht. Außerdem besteht die Aufgabe, ein Verfahren der eingangs genannten Art anzugeben, das einfach und kostengünstig durchführbar ist.

Bezüglich des Verfahrens wird die Aufgabe dadurch gelöst, dass an einer Teststelle auf einem Träger Rezeptoren immobilisiert werden, die mit den Uganden eine spezifische Bindung eingehen können, dass die Probe mit der Teststelle in Kontakt gebracht wird, dass mindestens ein die Häufigkeit der Bindungen zwischen den Liganden und den Rezeptoren repräsentierender erster Messwert erfasst wird, dass danach eine Sättigungslösung, welche die Liganden in höherer Konzentration enthält als die Probe, derart mit den Rezeptoren in Kontakt graucht wird, dass im Wesentlichen alle an der Teststelle befindlichen Rezeptoren an einen Liganden gebunden sind, dass danach ein die Häufigkeit der Bindungen zwischen den Liganden und den Rezeptoren repräsentierender zweiter Messwert erfasst wird, und dass mit Hilfe des ersten und zweiten Messwerts die Konzentration der Liganden in der Probe bestimmt wird.

In vorteilhafter Weise ist es dadurch möglich, bei beiden Messungen denselben Detektor zu verwenden, wobei die zweite Messung dazu dient, die Flächenbelegung der Teststelle mit den Liganden zu bestimmen. Die Rezeptoren können Nukleinsäuren oder Derivate davon (DNA, RNA, PNA, LNA, Oligonukleotide, Plasmide, Chromosomen), Peptide, Proteine (Enzym, Protein, Oligopeptide, zelluläre Rezeptorproteine und deren Komplexe, Peptidhormone, Antikörper und deren Fragmente), Kohlenhydrate und deren Derivate, insbesondere glykosylierte Proteine und Glycoside, Fette, Fettsäuren und/oder Lipide umfassen.

Bei einer bevorzugten Ausführungsform des Verfahrens weist die Probe mindestens zwei unterschiedliche Arten von Liganden auf, wobei jeder Ligandenartjewells eine Rezeptorart zugeordnet ist, deren Rezeptoren für die Liganden der betreffenden Ligandenart bindungsspezifisch sind, wobei auf dem Träger für jede dieser Rezeptorarten jeweils mindestens eine Teststelle eingerichtet wird, an der Rezeptoren der betreffenden Rezeptorart immobilisiert werden, wobei die Probe mit den Teststellen in Kontakt gebracht wird, wobei für jede Teststelle mindestens ein die Häufigkeit der Bindungen zwischen den Liganden und den Rezeptoren der Teststelle repräsentierender erster Messwert erfasst wird, wobei danach die Sättlgungslösung, welche die Uganden der einzelnen Ligandenarten jeweils in höherer Konzentration enthält als die Probe, derart mit den Teststellen in Kontakt gebraucht wird, dass im Wesentlichen alle an den Teststellen befindlichen Rezeptoren an einen Liganden gebunden sind, wobei zur Bestimmung der Flächenbelegung der Teststellen mit den Liganden für jede Rezeptorart jeweils ein zweiter Messwert erfasst wird, und wobei mit Hilfe der ersten und zweiten Messwerte für jede Ligandenart jeweils die Konzentration der Liganden in der Probe bestimmt wird. In vorteilhafter Weise kann also die Konzentration mehrerer unterschiedlicher, in der Probe enthaltener Liganden gleichzeitig bestimmt werden.

Die vorstehend genannte Aufgabe wird bei einem Verfahren zur Bestimmung der Konzentration von in einer zu untersuchenden Probe enthaltenen Liganden,

- wobei auf einem Träger an mindestens einer Teststelle Rezeptoren immobilisiert werden, die mit den Liganden eine spezifische Bindung eingehen können,
- wobei die Probe während einer ersten Zeitdauer mit der mindestens einen Teststelle in Kontakt gebracht und danach von dieser entfernt wird,
- wobei mindestens ein die Häufigkeit der Bindungen zwischen den Liganden und den Rezeptoren repräsentierender erster Messwert erfasst wird,
- wobei die Probe für mindestens eine weitere Zeitdauer mit der Teststelle in Kontakt gebracht und danach von dieser entfernt wird,
- wobei mindestens ein die Häufigkeit der Bindungen zwischen den Liganden und den Rezeptoren repräsentierender weiterer Messwert erfasst wird, und
- wobei mit Hilfe des ersten und des mindestens einen weiterer Messwerts die Konzentration der Liganden in der Probe bestimmt wird.

Es findet also eine Mehrfachexposition der Rezeptoren mit den Liganden statt. In vorteilhafter Welse ist es dadurch möglich, auch Liganden-Konzentrationen, die in einem mehrere Dekaden umfassenden Konzentrationsbereich liegen können, mit großer Präzision zu messen.

Bei einer vorteilhaften Ausgestaltung des Verfahrens, die eine Bestimmung der Konzentrationen von mindestens zwei in einer zu untersuchenden Probe enthaltenen Uganden unterschiedlicher Ligandenarten ermöglicht, von denen Liganden einer ersten Ligandenart eine höhere Konzentration aufweisen als Liganden einer zweiten Ligandenart, werden folgende Verfahrensschritte durchgeführt.

- dass auf einem Träger an einer ersten Teststelle Rezeptoren einer ersten Rezeptorart und an einer zweiten Teststelle Rezeptoren einer zweiten Rezeptorart immobilisiert werden,
- dass die Rezeptoren der ersten Rezeptorart mit den Uganden der ersten Ligandenart und die Rezeptoren der zweiten Rezeptorart mit den Liganden der zweiten Ligandenart jeweils eine spezifische Bindung eingehen können,
- dass die Probe während einer ersten Zeitdauer mit den Teststellen in Kontakt gebracht und danach von den Teststellen entfernt wird,
- dass mindestens ein die Häufigkeit der Bindungen zwischen den Liganden der ersten Ugandenart und den Rezeptoren der ersten Rezeptorart repräsentierender erster Messwert erfasst wird,
- dass die Probe für eine zweite Zeitdauer mit den Teststellen in Kontakt gebracht und danach von den Teststellen entfernt wird,
- dass mindestens ein die Häufigkeit der Bindungen zwischen den Liganden der zweiten Ligandenart und den Rezeptoren der zweiten Rezeptorart repräsentierender zweiter Messwert erfasst wird, und
- dass mit Hilfe der ersten und zweiten Messwerte die Konzentrationen der Liganden in der Probe bestimmt werden.

In vorteilhafter Weise können dabei die erste und zweite Zeitdauer so gewählt werden, dass die den einzelnen Ligandenarten zugeordneten Teststellen jeweils in einem günstigen Arbeitspunkt betrieben werden, in dem die Messkennlinie (Messwert als Funktion der Ligandenkonzentration) eine hohe Steigung aufweist. Somit ergibt sich eine entsprechend große Messempfindlichkeit. Durch die Mehrfachexposition kann der mit den Rezeptoren beschichtete Träger für mehrere zeitlich voneinander beabstandete Messungen verwendet werden. Das Verfahren ist also Material sparend. Bei der Bestimmung der Konzentration können zusätzlich zu den ersten und zweiten Messwerten vorgegebene Kenngrößen, die beispielsweise mit Hilfe eines baugleichen Sensorchips durch eine Kalibratlonsmessung bestimmt werden können, berücksichtigt werden.

Vorteilhaft ist, wenn

- die Teststellen nach dem Erfassen des zweiten Messwerts mit einer Sättigungslösung, welche die Liganden in höherer Konzentration enthält als die Probe, in Kontakt gebracht werden,
- wenn danach ein die Häufigkeit der Bindungen zwischen den Liganden der ersten Ligandenart und den Rezeptoren der ersten Rezeptorart repräsentierender dritter Messwert sowie ein die Häufigkeit der Bindungen zwischen den Liganden der zweiten Ligandenart und den Rezeptoren der zweiten Rezeptorart repräsentierender vierter Messwert erfasst werden, und
- wenn mit Hilfe der ersten, zweiten, dritten und vierten Messwerte die Konzentrationen der Liganden in der Probe bestimmt werden.

Mit Hilfe der dritten und vierten Messwerte kann die Flächenbelegung der Teststelle mit den Liganden und somit indirekt auch mit den Rezeptoren bestimmt und bei der Konzentrationsmessung berücksichtigt werden. Dadurch ergibt sich eine noch größere Messgenauigkeit, Insbesondere wenn die Flächenbelegung des Trägers mit den Rezeptoren beispielsweise aufgrund von Fertigungstoleranzen Schwankungen unterworfen ist.

Bei einer zweckmäßigen Ausführungsform der Erfindung

- wird die erste Zeitdauer derart gewählt, dass nur ein Teil der an der ersten Teststelle befindlichen Rezeptoren an einen Liganden binden,
- wobei die zweite Zeitdauer derart gewählt wird, dass an der ersten Teststelle im Wesentlichen alle Rezeptoren und an der zweiten Teststelle nur ein Teil der Rezeptoren an einen Liganden binden,
- wobei nach Ablauf der zweiten Zeitdauer mindestens ein die Häufigkeit der Bindungen zwischen den Liganden der ersten Ligandenart und den Rezeptoren der ersten Rezeptorart repräsentierender dritter Messwert erfasst wird, und
- wobei nach Ablauf der zweiten Zeitdauer mindestens eine Sattigungsiösung, welche die Liganden der zweiten Ligandenart in höherer Konzentration enthält als die Probe, derart zumindest mit den Rezeptoren der zweiten Rezeptorart in Kontakt gebraucht wird, dass im Wesentlichen alle an der zweiten Teststelle befindlichen Rezeptoren an einen Liganden gebunden sind,
- wobei danach ein die Häufigkeit der Bindungen zwischen den Liganden der zweiten Ligandenart und den Rezeptoren 5) der zweiten Rezeptorart repräsentierender vierter Messwert erfasst werden, und
- wobei mit Hilfe der ersten, zweiten, dritten und vierten Messwerte die Konzentrationen der Liganden (6) in der Probe bestimmt werden.

Das Verfahren ist dann noch einfacher durchführbar.
Bei einer zweckmäßigen Ausgestaltung der Erfindung werden in Abhängigkeit von der Bindung der Liganden an die Rezeptoren Luminophoren und/oder Chemiluminophoren direkt und/oder Indirekt über Nachweisantikörper an die Liganden gebunden, wobei die Luminophoren und/oder Chemilumlnophoren zur Abgabe von Lumineszenzstrahlung angeregt werden, und wobei die ersten und zweiten Messwerte durch Messung der Lumineszenzstrahlung erfasst werden. Das Verfahren kann also mit einem ELISA und/oder Sandwich-ELISA kombiniert werden.

Zur gleichzeitigen Messung der Konzentration mehrerer unterschiedlich konzentrierter Liganden ist es vorteilhaft, wenn auf dem Träger mindestens zwei Teststellen eingerichtet werden, auf denen Rezeptoren unterschiedlicher Rezeptorarten mit unterschiedlicher Dichte immobilisiert werden. Dabei werden die immobilisierungsdichten anhand von Erfahrungs- und/oder Schätzwerten für die Konzentrationen vorzugsweise derart aufeinander abgestimmt, dass die die unterschiedlichen immobllisierungsdlchten aufweisenden Teststellen während des Kontakts mit der Probe etwa gleich stark durch das Binden der Liganden an die Rezeptoren ausgesteuert werden.

Bei einer anderen zweckmäßigen Ausgestaltung des Verfahrens werden auf dem Träger mindestens zwei Teststellen eingerichtet auf denen Rezeptoren derselben Rezeptorart mit unterschiedlicher Dichte immobillisiert werden. Mit dem Verfahren kann dann über einen breiten Konzentrationsbereich eine hohe Messgenaulgkeit erzielt werden.

Bezüglich der Vorrichtung wird die vorstehend genannte Aufgabe dadurch gelöst, dass die Vorrichtung einen Träger aufweist, an dessen Oberfläche mindestens eine Teststelle angeordnet ist, an der Rezeptoren immobilisiert sind, die beim Kontaktieren der Uganden mit diesen eine spezifische Bindung eingehen, dass die Vorrichtung wenigstens einen Detektor zur Erfassung von Messwerten hat, die von der Häufigkeit der Liganden-Rezeptor-Bindungen abhängig sind, dass ein mit dem Detektor verbundenen Datenspeicher zum Zwischenspeichern eines ersten, nach dem inkontaktbringen der Probe mit der Teststelle erfassbaren Messwerts vorgesehen ist, dass die Vorrichtung ein Reservoir für eine die Liganden in hoher Konzentration enthaltende Sättigungslösung aufweist, dass eine Fördereinrichtung zum Zuführen der Sättlgungslösung aus dem Reservoir zu der mindestens einen Teststelle vorgesehen ist, und dass die Vorrichtung eine zumindest mit dem Datenspeicher und der Fördereinrichtung in Steuerverbindung stehenden Auswerte- und Steuereinrichtung aufweist, die derart ausgestaltet ist, dass nach dem Zwischenspelchem des ersten Messwerts die Söffigungslösung der Teststelle derart zugeführt wird, dass im Wesentlichen alle an der mindestens einen Teststelle befindlichen Rezeptoren an einen Liganden binden, und dass dann mit Hilfe des Detektors ein zweiter Messwert für die Flächenbelegung der Teststelle mit den Uganden erfasst und anhand des ersten und zweiten Messwerts ein Messsignal für die Konzentration der Liganden in der Probe erzeugt wird.

Die Vorrichtung ist also so ausgestaltet, dass mit Hilfe des Detektors nacheinander zunächst ein die Häufigkeit der Bindungen zwischen den Liganden und den Rezeptoren repräsentierender erster Messwert und danach ein zweiter Messwert für die Flächenbelegung der Teststelle mit den Liganden erfasst werden kann. Somit kann ein zusätzlicher Sensor zur Messung der Flächenbelegung eingespart werden. Die Vorrichtung kann auch für heterologe Assays verwendet werden. Dabei können kompetitive und nichtkompefitive Assays auf einem Träger durchgeführt werden. Ferner besteht die Möglichkeit, die Konzentrationen unterschiedlicher Molekülarten, wie z.B. Nukleinsäuren und Proteine, gleichzeitig zu messen.

Vorteilhaft ist, wenn zum Untersuchen einer mindestens zwei unterschiedliche Arten von Liganden aufweisenden Probe mehrere Teststellen an der Oberfläche des Trägers vorgesehen sind, an denen Rezeptoren immobilisiert sind, die für Uganden unterschiedlicher Ligandenarten bindungsspezifisch sind, wenn jeder Teststelle Jeweils ein mit dem Datenspeicher verbundener Detektor zugeordnet ist, wenn der Datenspeicher zumindest eine der Anzahl der Teststellen entsprechende Anzahl Speicherplätze zum Zwischenspeichern von ersten, nach dem inkontaktbringen der Probe mit der Teststelle erfassbaren Messwerten aufweist und wenn die Auswerte- und Steuereinrichtung derart ausgestaltet ist, dass nach dem Zuführen der Sättigungslösung zu den Teststellen mit Hilfe der Detektoren für Jede Teststelle jeweils ein zweiter Messwert für die Flächenbelegung der Teststelle mit den Liganden erfasst und anhand des ersten und zweiten Messwerts jeweils ein Messsignal für die Konzentration der für die Rezeptoren der betreffende Teststelle bindungsspeziflschen Liganden in der Probe erzeugt wird. Dadurch ist es möglich, auf einem einzigen Träger mit nur einem Versuch die Konzentrationen mehrerer Liganden gleichzeitig zu messen. Dabei sind die Flächenbelegungsdlchten an den einzelnen Teststellen bevorzugt so auf die bei der Messung zu erwartenden Ligandenkonzentrationen abgestimmt, dass die an den einzelnen Teststellen befindlichen Detektoren bei einer zeitgleichen Erfassung der ersten Messwerte etwa gleich weit ausgesteuert werden. Die Flächenbelegungsdichte einer Rezeptorart, die zur Bestimmung der Konzentration einer Ligandenart vorgesehen ist, deren Liganden in hoher Konzentration in der Probe enthalten sind, kann dabei kleiner sein, als die Flächenbelegungsdlchte einer Rezeptorart, die zur Bestimmung der Konzentration einer Ligandenart vorgesehen ist, deren Liganden in geringerer Konzentration in der Probe enthalten sind.

Die vorstehend genannte Aufgabe wird auch durch eine Vorrichtung gelöst, die einen Träger aufweist, an dessen Oberfläche mindestens eine Teststelle angeordnet ist, an der Rezeptoren immobilisiert sind, die beim Kontaktieren der Liganden mit diesen eine spezifische Bindung eingehen, mit wenigstens einem Detektor zur Erfassung von Messwerten, die von der Häufigkeit der Liganden-Rezeptor-Bindungen abhängig sind, wobei die Vorrichtung einen mit dem Detektor verbundenen Datenspeicher zum Zwischenspeichern zumindest eines ersten, nach dem Inkontaktbringen der Probe mit der Teststelle erfassbaren Messwerts hat, und wobei die Vorrichtung eine mit dem Reservoir und der Teststelle verbundene Fördereinrichtung aufweist, mit dem die Probe von dem Reservoir zu der Teststelle und von der Teststelle zu dem Reservoir förderbar ist.

Die Probe kann dann auf einfache Weise mehrfach mit der Teststelle In Kontakt gebracht und ggf wieder von dieser entfernt werden, um nacheinander mehrere Messungen vorzunehmen. Dadurch kann für die einzelnen Rezeptorarten jeweils ein günstiger Arbeitspunkt gewählt werden.

Bei einer bevorzugten Ausgestaltung der Erfindung weist die Vorrichtung zusätzlich zu dem ersten Reservoir mindestens ein zweites Reservoir auf, in dem ein mit einem optischen Marker markierter oder markierbarer Nachweisantikörper angeordnet ist, der dazu geeignet Ist, an die Liganden zu binden, wobei der Marker mittels der Fördereinrichtung der mindestens einen Teststelle zuführbar ist, und wobei der Detektor als optischer Detektor zum Erfassen einer bel Anwesenheit des Markers auftretenden Lumineszenzstrahlung ausgebildet ist. Mit der Vorrichtung kann dann auf einfache Weise ein ELISA und/oder Sandwich-ELISA durchgeführt werden.

Vorteilhaft ist, wenn die Vorrichtung ein drittes Reservoir aufweist, in dem ein Cheml-Luminophor angeordnet ist, der bei einem Kontakt mit dem Marker zur Abgabe einer Cheml-Lumineszenzstrahlung anregbar ist, und wenn der Chemi-Luminophor mittels der Fördereinrichtung der mindestens einen Teststelle zuführbar ist. Die Bindung der Liganden an die Rezeptoren kann dann mit Hilfe von Chemi-Lumineszenz nachgewiesen werden. Als Chemi-Luminophor ist bevorzugt Luminol In dem dritten Reservoir bevorratet.

Bei einer zweckmäßigen Ausgestaltung der Erfindung weist die Vorrichtung mindestens ein viertes Reservoir auf, in dem eine Spülflüssigkeit angeordnet ist, wobei die Spülflüssigkeit mittels der Fördereinrichtung der mindestens einen Teststelle zuführbar ist. Nachdem die Probe für eine vorgegebene Zeitdauer mit der Teststelle in Kontakt gebracht wurde, kann dann die Teststelle mittels der Spülflüssigkeit gespült werden, um nicht an einen Rezeptor gebundene Liganden aus dem Detektionsbereich des Detektors zu entfernen. In entsprechender Weise kann ein weiterer Spülschritt durchgeführt werden, nachdem die Teststelle für eine vorgegebene Zeitdauer mit den Nachweisantikörpern in Kontakt gebracht wurde. Dabei werden Nachweisantikörper, die nicht an einen Rezeptor-Llganden-Komplex gebunden sind von der Teststelle entfernt.

Vorteilhaft ist, wenn die Vorrichtung ein fünftes Reservoir aufweist, in dem Wasserstoffperoxid angeordnet ist, und wenn das Wasserstoffperoxid mittels der Fördereinrichtung der mindestens einen Teststelle zuführbar ist. Dabei kann als Marker ein Peroxidase-Enzym, vorzugsweise Meerrettich-Peroxidase, und als Luminophor Luminol vorgesehen sein. Wenn die Meerrettich-Peroxidase mit dem Wasserstoffperoxid In Kontakt gerät, werden freie Sauerstoffradikale von dem Wasserstoffperoxid abgespalten, wobei das Luminol unter Abgabe von Lumineszenzstrahlung chemisch zersetzt wird.

Bei einer vorteilhaften Ausgestaltung der Erfindung sind das erste und zweite Reservoir sowie ggf. das dritte und/oder vierte Reservoir durch mit Flüssigkeiten befüllte, hintereinander angeordnete Abschnitte einer Kapillare gebildet sind, die an einem Abgabeende mit einer die mindestens eine Teststelle aufweisenden Messkammer verbunden Ist, und dass die in den Reservoiren gespeicherten Flüssigkeiten mittels der Fördereinrichtung entlang der Kapillare In die Messkammer verschiebbar sind. Die einzelnen, in der Kapillare enthaltenen Flüssigkeiten können dann auf einfache Weise, in der Reihenfolge, In der sie In der Kapillare hintereinander angeordnet sind, mit der mindestens einen Teststelle in Kontakt gebracht werden. Durch den relativ geringen Querschnitt der Kapillare wird eine Vermischung der Flüssigkeiten in der Kapillare weitgehend vermieden.

Bei einer bevorzugten Ausführungsform der Erfindung ist zwischen zueinander benachbarten Reservoiren in der Kapillare Jeweils eine Diffusionssperre angeordnet, die vorzugsweise durch eine hydrophobe Flüssigkeit gebildet ist Dadurch wird vermieden, dass sich die unterschiedlichen, in den einzelnen Abschnitten der Kapillare enthaltenen Flüssigkeiten durch Diffusion miteinander vermischen. Als Diffusionssperre kann auch ein Gas und/oder Gasgemisch dienen, Insbesondere Luft.

Vorteilhaft ist, wenn die Vorrichtung zusätzlich zu der ersten Kapillare mindestens eine zweite Kapillare aufweist, die mit dem Wasserstolfperoxld befüllt ist, und wenn die erste Kapillare und die zweite Kapillare Jeweils mit einer Einlassöffnung eines Mischers verbunden sind, der mit einer Auslassöffnung an der Messkammer angeschlossen ist. Das Wasserstoffperoxid ist also in einer separaten zweiten Kapillare angeordnet und wird erst unmittelbar, bevor es zu der Teststelle geleitet wird, mit dem Luminophor gemischt. Somit kann das relativ aggressive Wasserstoffperoxid bei einer Lagerung der Vorrichtung nicht mit den In der ersten Kapillare enthaltenen Flüssigkeiten In Kontakt geraten und diese schädigen.

Bei einer zweckmäßigen Ausgestaltung der Erfindung weist die Fördereinrichtung mindestens eine mit einem fließfähigen Medium befüllte Druckerzeugungskammer auf, die zum Verdrängen der in der Kapillare bevorrateten Flüssigkeiten mit dem von der Messkammer entfernten Ende der Kapillare verbunden ist, wobei die Druckerzeugungskammer eine Einrichtung zum Erzeugen eines hydraulischen und/oder pneumatischen Drucks in dem fließfähigen Medium aufweist. Der Inhalt der Kapillare kann dann auf einfache Weise durch Anlegen eines Überdrucks in der Druckerzeugungskammer in die Messkammer entleert werden.

Vorteilhaft Ist, wenn die Einrichtung zum Erzeugen des hydraulischen und/oder pneumatischen Drucks eine Heizeinrichtung zum Beheizen des in der Druckerzeugungskammer befindlichen fließfähigen Mediums aufweist. Beim Erwärmen dehnt sich dann das fließfähige Medium aus, wodurch ein Überdruck entsteht, der die in der Kapillare gespeicherten Flüssigkeiten in die Messkammer verschiebt. Das fließfähige Medium kann eine Flüssigkeit oder ein Gas sein, beispielsweise Luft.

Bei einer anderen Ausführungsform der Erfindung weist die Druckerzeugungskammer eine verformbare Kammerwand auf, die durch Druckbeaufschlagung an Ihrer Außenseite in eine Innenhöhlung der Druckerzeugungskammer auslenkbar ist. Der hydraulische und/oder pneumatische Druck kann dann vom Benutzer der Vorrichtung durch Druckbeaufschlagung der verformbaren Kammerwand beispielsweise mit einem Finger erzeugt werden.

Vorteilhaft ist, wenn die Messkammer eine mit einem Aufnahmebehälter verbundene Auslassöffnung hat, und wenn in dem Aufnahmebehälter ein saugfähiges Medium angeordnet ist. Sobald die Flüssigkeit mit dem saugfähigen Medium in Kontakt gerät, saugt dieses weitere Flüssigkeit an, wodurch sich eine Strömung aufbaut, die von der Kapillare durch die Messkammer zu dem Aufnahmebehälfier verläuft, zum Entlüften des Aufnahmebehälter kann dieser ein Überdruckventil aufweisen.

Nachfolgend ist ein Ausführungsbeispiel der Erfindung anhand der Zeichnung näher erläutert. Es zeigen zum Teil stärker schematisiert:

Fig. 1a     eine Aufsicht auf ein erstes Ausführungsbeispiel einer Vorrichtung zur Bestimmung der Konzentration von in einer zu untersuchenden Probe enthaltenen Liganden,

Fig. 1b     eine Aufsicht auf ein zweites Ausführungsbeispiel der Vorrichtung,

Fig. 1c     eine Aufsicht auf ein drittes Ausführungsbeispiel der Vorrichtung,

Fig. 2     einen Querschnitt durch einen Detektor zur Erfassung von Lumineszenzstrahlung, wobei an der Oberfläche des Detektors ein Rezep-torLiganden-Komplex schematisch darge-stellt ist,

Fig. 3     eine graphische Darstellung der Konzentration S der an der Oberfläche immobilisierten Rezeptor-Liganden-Komplexe In Abhängigkeit von der Zeit T für zwei Teststellen und jeweils zwei unterschiedlichen Immobilisierungsdichten,

Fig.4     eine Aufsicht auf ein zweites Ausführungsbeispiel einer Vorrichtung zur Bestimmung der Konzentration von in einer zu untersuchenden Probe enthaltenen Liganden,

Fig. 5    einen Querschnitt durch das zweite Ausführungsbeispiel der Vorrichtung, und

Fig. 6    einen Querschnitt durch ein drittes Ausführungsbeispiel der Vorrichtung.

Eine im Ganzen mit 1 bezeichnete Vorrichtung zur Bestimmung der Konzentration von In einer zu untersuchenden flüssigen Probe enthaltenen Liganden weist einen Träger auf, der ein Gehäuseteil 2 aus einem biokompatiblen Werkstoff hat, an dem ein Halbleiterchip 3 angeordnet ist. An der Oberfläche des Halbleiterchips 3 sind mehrere Teststellen matrixförmig angeordnet.

An den einzelnen Teststellen ist Jeweils ein optischer Detektor 3 in die Oberfläche des Trägers eingelassen, auf dem Rezeptoren 5 immobilisiert sind, die beim Kontaktieren der Liganden 6 mit diesen eine spezifische Bindung eingehen. Der Halbleiterchip 3 grenzt mit den Teststellen an eine Messkammer 7 an, die eine mit einer Abdeckung 8 verschließbare Öffnung zum Einfüllen der Probe aufweist

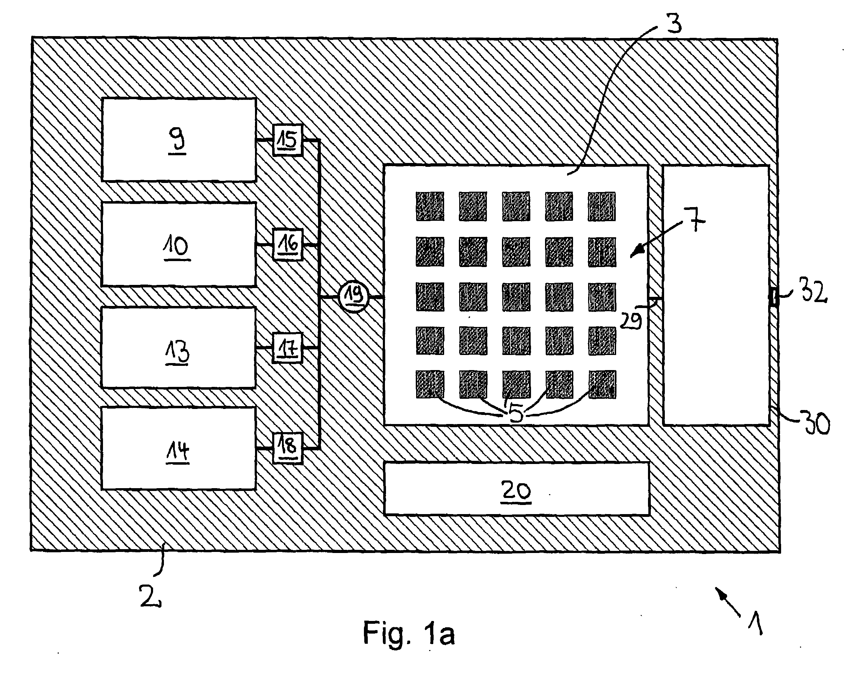

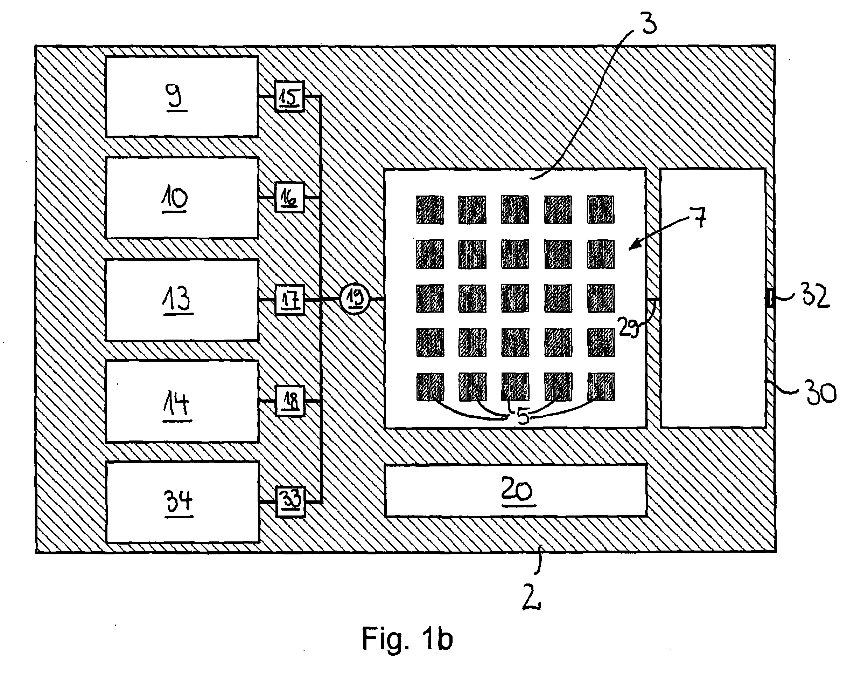

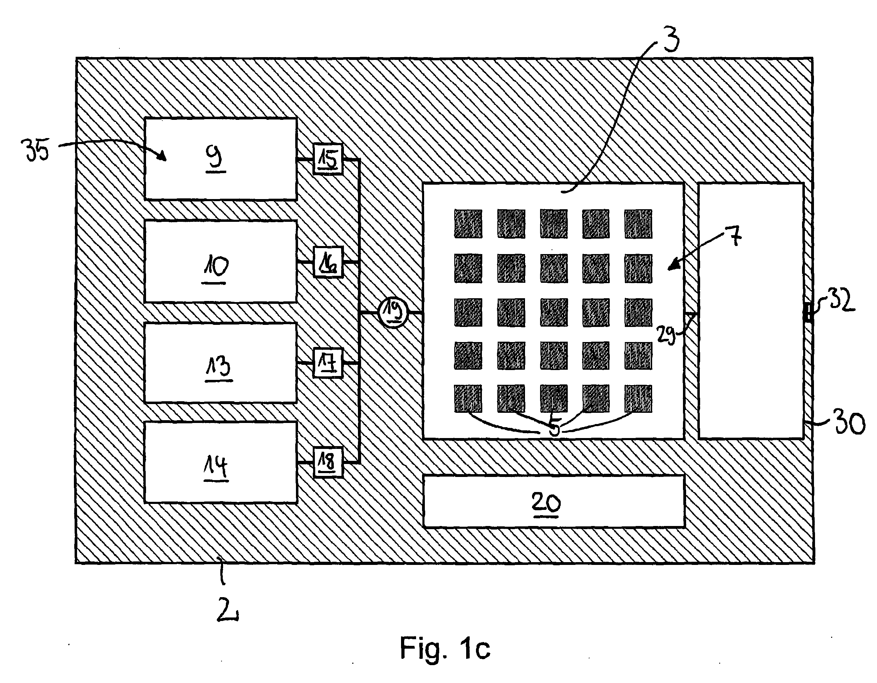

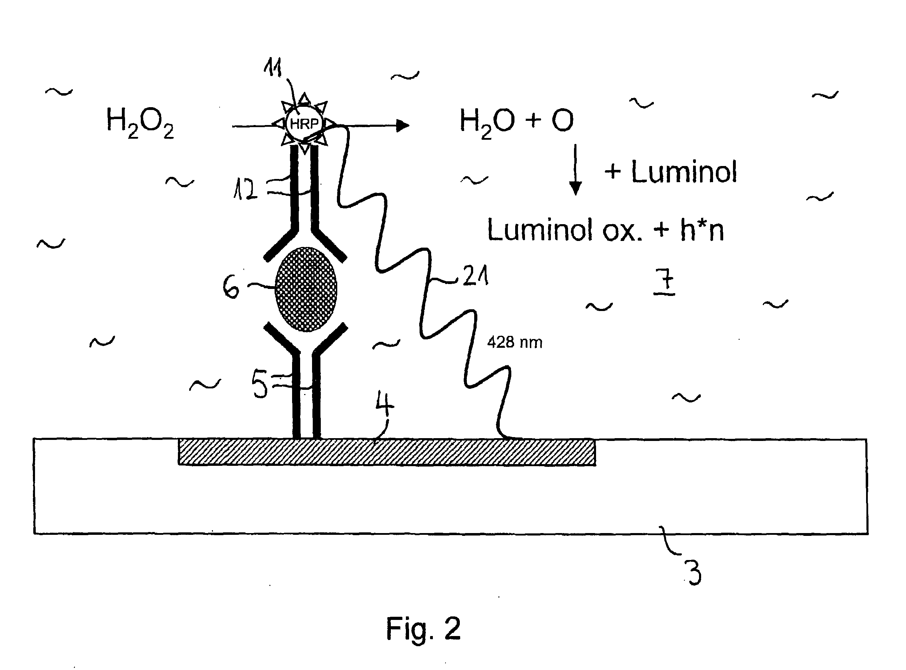

Bei dem in Fig. 1a, 1b und 2 gezeigten Ausführungsbeispiel hat die Vorrichtung 1 ein erstes Reservoir 9 für eine die Liganden in hoher Konzentration enthaltende Sättigungslösung. Ein zweites Reservoir 10 der Vorrichtung 1 ist mit einer Flüssigkeit befüllt, die mit einem optischen Marker 11, nämlich Meerrettich-Peroxidase (HRP), markierte Nachweisantikörper 12 enthält. Die Nachweisantikörper 12 sind jeweils für einen bestimmten Liganden 6 bindungsspezifisch.

Femer weist die Vorrichtung ein drittes Reservoir 13 auf, In dem eine Flüssigkeit angeordnet ist, die einen Chemi-Luminophor, nämlich Luminol, und Wasserstoffperoxid enthält. In einem vierten Reservoir 14 ist eine Spülflüssigkeit angeordnet, nämlich eine phosphatgepufferte Salzlösung (PBS - phosphat buffered saline solution). Die Spülflüssigkeit kann geeignet Hilfsstoffe, wie z.B. Tenside enthalten. Die Reservoirs 9, 10, 13, 14 sind jeweils als Innenhöhlungen In das Gehäuseteil 2 integriert.

Wie in Fig. 1a und 1b erkennbar ist, weist die Vorrichtung eine Fördereinrichtung auf, die Ventile 15, 16, 17, 18 und eine Pumpe 19 hat, die in den Halbleiterchip 3 integriert sein können. Die Reservoirs 9, 10, 13, 14 sind jeweils über ein ihnen zugeordnetes Ventil 15, 16, 17, 18 mit einem ersten Anschluss der Pumpe 19 verbunden. Ein zweiter Anschluss der Pumpe 19 ist an einer ersten Öffnung der Messkammer 7 angeschlossen. Die Ansteuerung der Ventile 15, 16, 17, 18 und der Pumpe 19 erfolgt mittels einer Auswerte- und Steuereinrichtung 20, die über In der Zeichnung nicht näher dargestellte elektrische Verbindungsleitungen mit den Ventilen 15, 16, 17, 18 und der Pumpe 19 in Steuerverbindung steht.

Zur Bestimmung der Konzentrationen der einzelnen Liganden 6 in der Probe wird die Probe zunächst auf die Teststellen aufgebracht. Das Aufbringen der Probe kann mit Hilfe einer in der Zeichnung nicht näher dargestellten Pipette durch die Öffnung der Messkammer 7 hindurch erfolgen. Danach wird die Messkammer 7 mit der Abdeckung 8 verschlossen.

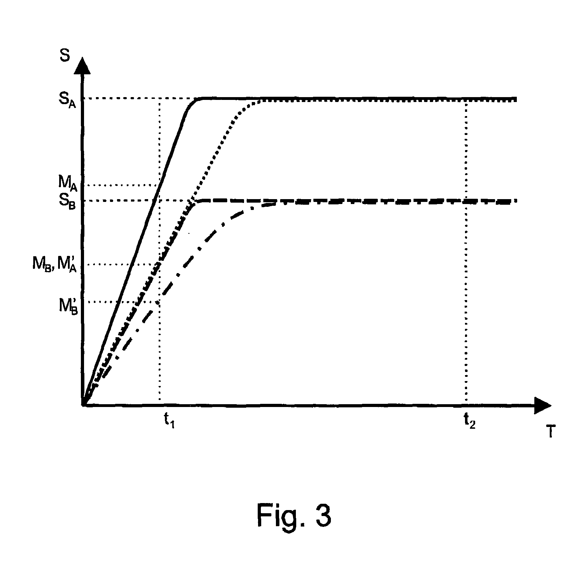

Nachdem die Probe mit den Teststellen In Kontakt gebracht wurde, wird eine Zeitdauer $t_1$ abgewartet, um den Liganden 6 Gelegenheit zu geben, spezifisch an die Rezeptoren 5 zu binden. In Fig. 3 sind die Konzentrationen der an der Oberfläche immobilisierten Rezeptor-Liganden-Komplexe in Abhängigkeit von der Zeitdauer 6 mit der betreffenden Teststelle In Kontakt stehen, für zwei mit unterschiedlichen Rezeptoren 5 beschichtete Teststellen jeweils für zwei unterschiedliche immobilislerungsdichten graphisch dargestellt. Dabei markiert die durchgezogene Line den Verlauf des Messsignals an einer mit ersten Rezeptoren 5 beschichteten Teststelle, für den Fall, dass die ersten Rezeptoren 5 eine erste immobilisierungsdichte aufweisen und mit einer Probe in Kontakt stehen, welche für die ersten Rezeptoren 5 bindungsspezifische erste Liganden in einer ersten Konzentration enthält. Deutlich ist erkennbar, dass das Messsignal zunächst relativ steil ansteigt, bis es eine Sättigungskonzentration $S_A$ erreicht. Die punktierte Line zeigt den Verlauf des Messsignals an der ersten Teststelle für den Fall, dass die ersten Rezeptoren 5 mit einer Probe in Kontakt stehen, welche die ersten Liganden in einer zweiten Konzentration aufweist, die geringer ist als die erste. Der Anstieg des Messsignals ist dabei geringer als bei der ersten Ligandenkonzentration, jedoch erreicht das Messsignal dieselbe Sättigungskonzentration $S_A$.

Die strichlinierte Linie zeigt den Messsignalverlauf für eine mit zweiten Rezeptoren 5 beschichtete Teststelle für den Fall, dass die zweiten Rezeptoren 5 die erste Immobilisierungsdichte aufweisen und mit einer Probe in Kontakt stehen, welche für die zweiten Rezeptoren 5 bindungsspezifische zweite Liganden in der ersten Konzentration enthält Das Messsignal steigt bis zum Erreichen einer Sättigungskonzentration $S_B$, die geringe ist als die Sättigungskonzentration $S_A$ an. Die strichpunktierte Line zeigt den Verlauf des Messsignals an der zweiten Teststelle für den Fall, dass die zweiten Rezeptoren 5 mit einer Probe in Kontakt stehen, welche die zweiten Liganden in der zweiten Konzentration enthält. Der Anstieg des Messsignals ist geringer als bei der ersten Konzentration der zweiten Liganden, jedoch erreicht das Messsignal dieselbe Sättigungskonzentration $S_B$.

Die Zeitdauer $t_1$ wird so gewählt, dass die den einzelnen Teststellen zugeordneten Konzentrationen $M_A$, $M_B$, $M'_A$, $M'_B$, zum Zeitpunkt $t_1$ jeweils kleiner sind als die Söttigungskonzentration $S_A$, $S_B$ der entsprechenden Teststelle. Die Zeitdauer $t_1$ kann anhand eines zuvor ermittelten Schätzwerts für die Konzentrationen bestimmt werden.

Nachdem die Probe während der Zeitdauer $t_1$ mit den Rezeptoren 5 in Kontakt stand, werden nicht an einen Rezeptor 5 gebundene Bestandteile der Probe mit Hilfe der Spülflüssigkeit von den Teststellen entfernt so dass dann nur noch diejenigen Liganden 6 an den Teststellen angeordnet sind, die spezifisch an einen Rezeptor 5 gebunden sind. Zum Spülen der Teststellen wird das Ventil 18 geöffnet und die Spülflüssigkeit mittels der Pumpe 19 aus dem ersten Reservoir 9 in die Messkammer 7 gefördert. Die Ventile 15, 16, 17 sind geschlossen.

In einem weiteren Verfahrensschritt werden die an den Teststellen verbliebenen Liganden 6 durch Schließen des Ventils 18 und Öffnen des Ventils 16 mit den mit dem Marker 11 markierten Nachweisantikörpern 12 in Kontakt gebracht. Diese binden an die Liganden 6, nichtjedoch an freie Rezeptoren 5.

Nun werden eventuell noch vorhandene freie Marker-Nachwelsantikörper-Komplexe durch einen weiteren Spülschritt von den Teststellen entfernt. Die Marker 11 sind jetzt nur noch an den Stellen vorhanden, an denen ein Ugand 6 an einen an dem Halbleiterchip 3 immobilisierten Rezeptor 5 gebunden ist. Die Rezeptor-Liganden-Komplexe sind also mit dem Marker 11 markiert.

Danach wird in entsprechender Welse die in dem dritten Reservoir 13 befindliche, das Wasserstoffperoxid und den Chemi-Luminophor enthaltende Flüssigkeit mit den Teststellen in Kontakt gebracht. Eine solche Flüssigkeit ist beispielsweise unter der Bezeichnung "ECL®-Lösung" im Handel erhältlich. Wenn die Meerrettich-Peroxidase 9 mit dem Wasserstoffperoxid In Kontakt gerät, werden freie Sauerstoffradikale von dem Wasserstoffperoxid abgespalten, wodurch der Chemi-Luminophor unter Abgabe von Lumineszenzstrahlung 21 chemisch zersetzt wird. Es wird also in Abhängigkeit von der Bindung des Liganden 6 an den Rezeptor 5 eine Lumineszenzstrahlung 20 erzeugt. Für die einzelnen Teststellen wird jeweils ein erster Messwert $M_1$ für die Lumineszenzstrahlung 20 mit Hilfe des an der betreffenden Teststelle befindlichen Detektors 4 erfasst. Die so erhaltenen Messwerte werden in einem Datenspeicher abgelegt.

Danach wird eine Sättigungstösung, welche die Liganden in höherer Konzentration enthält als die Probe, derart mit den Rezeptoren in Kontakt gebraucht, dass Im Wesentlichen alle an den Teststellen befindlichen Rezeptoren an einen Liganden gebunden sind. Dazu wird das Ventil 15 für das erste Reservoir 9 geöffnet und die Sättigungslösung mittels der Pumpe 19 aus dem ersten Reservoir 9 in die Messkammer 7 gefördert. Die Konzentration der Liganden in der Sättigungslösung ist wesentlich größer als die Konzentration der Liganden in der Probe, so dass bereits kurz nach dem inkontaktbringen der Söttigungstösung mit den Teststellen die Sättigungskonzentration $S_A$, $S_B$ erreicht wird. Nach dem Inkontaktbringen der Sättigungslösung mit den Teststellen wird eine vorgegebene Zeitdauer abgewartet, um sicherzustellen, dass an allen Teststellen die Sättigungskonzentration $S_A$, $S_B$ erreicht wurde. Danach wird zur Bestimmung der Flächenbelegung der einzelnen Teststellen mit den Liganden 6 für jede Teststelle jeweils ein zweiter Messwert erfasst. Mit Hilfe des ersten Messwerts $M_1$ und des zweiten Messwerts $M_2$ sowie einer bekannten Bindungskonstanten K wird für jede Teststelle die Ligandenkonzentration L wie folgt berechnet:

$$L = K \frac{M_1}{M_2}$$

Bei dem in Fig. 1b gezeigten Ausführungsbeispiel ist die Pumpe 19 derart ausgestaltet, dass sie in zueinander entgegengesetzte Richtungen fördern kann. Die Pumpe 19 Ist über ein Absperrelement 33 mit einem Zwischenspeicher 34 verbindbar. Dadurch ist es möglich, die in der Messkammer befindliche Probe aus der Messkammer 7 in den Zwischenspeicher 34 abzupumpen, um sie zu einem späteren Zeitpunkt durch Zurückpumpen in die Messkammer 7 erneut mit den Rezeptoren 5 In Kontakt zu bringen. Eine solche Mehrfachexposition der Probe ist insbesondere bei Proben vorteilhaft, die mindestens zwei die stark unterschiedlich konzentrierte Liganden 6 aufweisen, deren Konzentration gemessen werden soll.

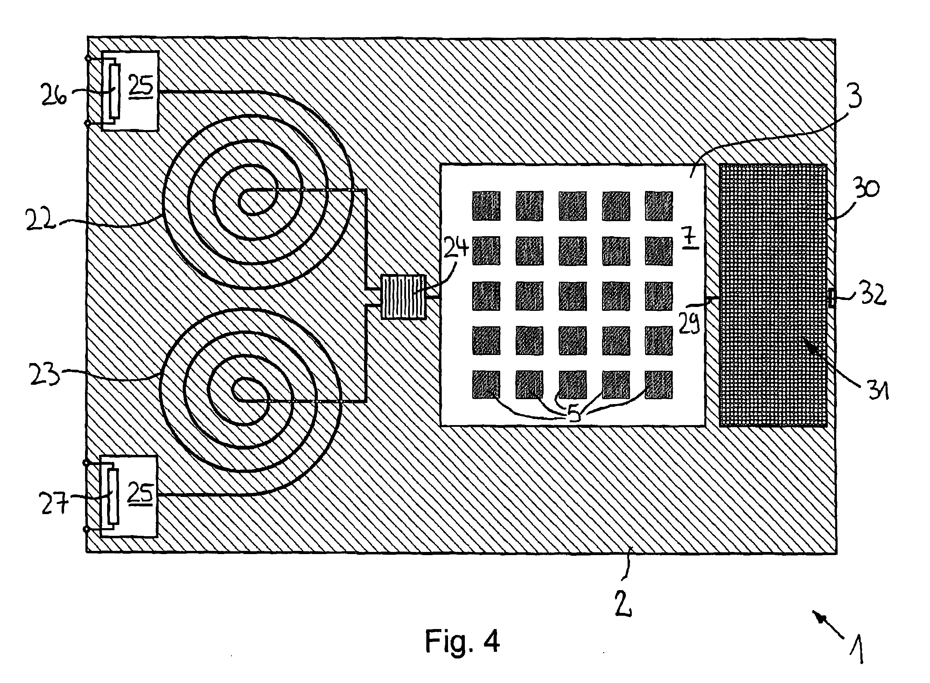

Bei den in Flg. 4 bis 6 dargestellten Ausführungsbeispielen sind die Reservoire durch Kapillarenabschnitte von spiralförmigen verlaufenden Kapillaren 22, 23 gebildet In einer ersten Kapillare 22 sind hintereinander eine erste Spülflüssigkeit, eine Nachweisantikörper enthaltende Flüssigkeit, eine zweite Spülflüssigkeit, eine einen Chemi-Luminophor enthaltende Flüssigkeit und eine die Liganden 6 In hoher Konzentration enthaltende Söttigungslösung angeordnet. Zwischen den unterschiedlichen Flüssigkeiten bzw. Lösungen ist jeweils eine hydrophobe Flüssigkeit angeordnet, die als Diffusionssperre dient. Die hydrophobe Flüssigkeit kann beispielsweise ein Öl sein. Eine zweiten Kapillare 23 ist mit Wasserstoffperoxid befüllt

Die Kapillaren 22, 23 sind jeweils mit einem Abgabeende mit einer Einlassöffnung eines Mischers 24 verbunden, der mit einer Auslassöffnung an der Messkammer 7 angeschlossen ist. Bei den in Fig. 4 bis 6 gezeigten Ausführungsbeispielen ist der Mischer 24 ein Möblus-Mischer, der derart ausgestaltet ist, dass die miteinander zu vermischenden, in der ersten Kapillare 22 und der zweiten Kapillare 23 enthaltenen Flüssigkeiten an den Wänden des Mischers 24 abwechselnd in zueinander entgegengesetzte Richtungen abgelenkt werden.

Bei dem in Fig. 1c gezeigten Ausführungsbeispiel hat die Vorrichtung 1 ein erstes Reservoir 9 mit einer Einlassöffnung 35, durch welche die Probe beispielsweise mit Hilfe einer Pipette in das erste Reservoir 9 eingefüllt werden kann. Ein zweites Reservoir 10 ist mit einer Flüssigkeit befüllt, die mit einem optischen Marker 11 markierte Nachweisantlkörper 12 enthält. Die Nachweisanflkörper 12 sind jeweils für einen bestimmten Liganden 6 blndungsspezifisch. In einem dritten Reservoir 13 ist eine Flüssigkeit angeordnet, die einen Chemi-Luminophor enthält. In einem vierten Reservoir 14 ist eine Spülflüssigkeit bevorratet.

Die Reservoirs sind wie bei dem Ausführungsbeispiel nach Fig.1a über Ventile 15, 16, 17, 18 und eine Pumpe 19 mit einer ersten Öffnung der Messkammer 7 verbunden. Die Ansteuerung der Ventile 15, 16, 17, 18 und der Pumpe 19 erfolgt mittels einer Auswerte- und Steuereinrichtung 20, wie nachstehend beschrieben.

Zur Bestimmung der Konzentrationen der einzelnen Liganden 6 in der Probe wird die Probe zunächst in das erste Reservoir 9 eingefüllt und bei geöffnetem Ventil 15 mit Hilfe der Pumpe 19 in die Messkammer 7 gefördert.

Nachdem die Probe mit den Teststellen in Kontakt gebracht wurde, wird eine erste Zeitdauer abgewartet, um den Liganden 6 Gelegenheit zu geben, spezifisch an die Rezeptoren 5 zu binden. Danach werden nicht an einen Rezeptor 5 gebundene Bestandteile der Probe von den Teststellen entfernt indem mit Hilfe der Pumpe 19 bei geöffnetem Ventil 18 und geschlossenen Ventilen 15, 16, 17 Spülflüssigkeit aus dem Reservoir 14 in die Messkammer 7 gefördert wird. Die in der Messkammer 7 befindliche Probe wird dadurch in einen Aufnahmebehälter 30 verdängt, der an einer zweiten Öffnung der Messkammer 7 angeschlossen ist.

In einem weiteren Verfahrensschritt werden die an einen Rezeptor 5 gebundenen Liganden 6 durch Schließen des Ventils 18 und Öffnen des Ventils 16 mit den mit dem Marker 11 markierten Nachweisantikörpern 12 in Kontakt gebracht.

Nun werden eventuell noch vorhandene freie Marker-Nachweisantikörper-Komplexe durch einen weiteren Spülschritt von den Teststellen entfernt. Die Marker 11 sind Jetzt nur noch an den Stellen vorhanden, an denen ein Ligand σ an einen an dem Halbleiterchip 3 immobilisierten Rezeptor 5 gebunden ist.

Danach wird in entsprechender Welse die In dem dritten Reservoir 13 befindliche, das Wasserstoffperoxid und den Chemi-Luminophor enthaltende Flüssigkeit mit den Teststellen in Kontakt gebracht. Wenn die Meerrettich-Peroxidase 9 mit dem Wasserstoffperoxid in Kontakt gerät, wird der Chemi-Luminophor unter Abgabe von Lumineszenzstrahlung 21 chemisch zersetzt. Für die einzelnen Teststellen wird Jeweils ein erster Messwert für die Lumineszenzstrahiung 20 mit Hilfe des an der betreffenden Teststelle befindlichen Detektors 4 erfasst. Die so erhaltenen Messwerte werden in einem Datenspeicher abgelegt.

Nun wird die Probe erneut mit den Teststellen in Kontakt gebracht, indem eine In dem ersten Reservoir 9 noch befindliche Probenmenge bei geöffnetem Ventil 15 und geschlossenen Ventilen 16, 17, 18 mit Hilfe der Pumpe 19 in die Messkammer 7 gefördert wird. Dann wird eine zweite Zeitdauer abgewartet, um den Liganden 6 Gelegenheit zu geben, spezifisch an die noch freien Rezeptoren 5 zu binden. Danach werden nicht an einen Rezeptor gebundene Bestandteile der Probe von den Teststellen entfernt, Indem mit Hilfe der Pumpe 19 bei geöffnetem Ventil 18 und geschlossenen Ventilen 15, 16, 17 erneut Spülflüssigkeit aus dem Reservoir 14 in die Messkammer 7 gefördert wird.

In einem weiteren Verfahrensschritt werden die Teststellen durch Schließen des Ventils 18 und Öffnen des Ventils 16 mit den mit dem Marker 11 markierten Nachwelsantlkörpem 12 in Kontakt gebracht. Nun werden eventuell noch vorhandene freie Marker-Nachweisantikörper-Komplexe durch einen Spülschritt von den Teststellen entfernt.

Dann wird für die einzelnen Teststellen jeweils ein zweiter Messwert für die Lumineszenzstrahlung 20 mit Hilfe des an der betreffenden Teststelle befindlichen Detektors 4 erfasst. Mit Hilfe der ersten und zweiten Messwerte sowie zuvor ermittelten Kalibrationskonstanten werden die Konzentrationen der Liganden 6 in der Probe bestimmt.

Bei den In Fig. 4 bis 6 dargestellten Ausführungsbeispiele weist die Fördereinrichtung für jede Kapillare 22, 23 jeweils eine mit einem fließfähigen Medium befüllte, bis auf einen Auslass geschlossene Druckerzeugungskammer auf, die an dem Auslass mit den von der Messkammer entfernten Enden der Kapillare verbunden ist. Die Druckerzeugungskammer hat eine Einrichtung zum Erzeugen eines hydraulischen und/oder pneumatischen Drucks in dem fließfähigen Medium.

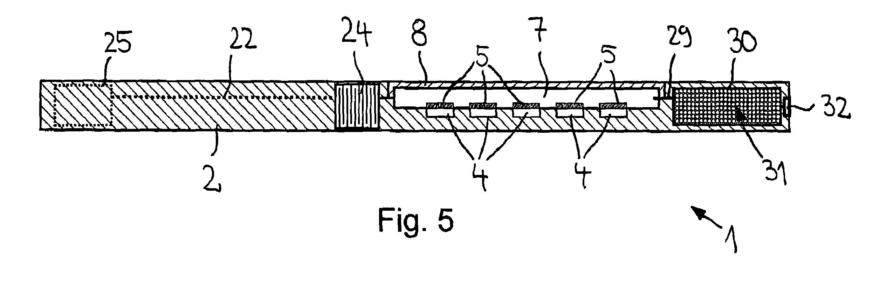

Bei dem in Fig. 4 und 5 gezeigten Ausführungsbeispielen hat das Medium einen positiven Temperaturkoeffizienten, d.h. es dehnt sich bei Erwärmung aus. Die Einrichtung zum Erzeugen des hydraulischen und/oder pneumatischen Drucks weist für jede Druckerzeugungskammer jeweils eine elektrische Heizeinrichtung 26, 27 zum Beheizen des Mediums auf Eine der ersten Kapillare 22 zugeordnete erste Heizeinrichtung 26 wird zum Verdrängen der in der ersten Kapillare gespeicherten Flüssigkeiten in die Messkammer 7 über die Auswerte- und Steuereinrichtung 20 eingeschaltet, nachdem die Probe mit den Teststellen in Kontakt gebracht wurde. Dadurch werden die in der ersten Kapillare 22 gespeicherten Flüssigkeiten, in der Reihenfolge, in der sie in der Kapillare 22 hintereinander angeordnet sind, in die Messkammer 7 abgegeben.

Eine der zweiten Kapillare 23 zugeordnete zweite Heizeinrichtung 26 wird später eingeschaltet als die erste Heizeinrichtung 26. Der Einschaltzeitpunkt wird so gewählt, dass das in der zweiten Kapillare 23 gespeicherte Wasserstoffperoxid in dem Mischer 24 mit der den Chemiluminophor enthaltenden Flüssigkeit gemischt wird. Die übrigen, in der ersten Kapillare 22 gespeicherten Flüssigkeiten geraten, bevor sie der Mischkammer 7 zugeführt werden, praktisch nicht mit dem Wasserstoffperoxid In Kontakt.

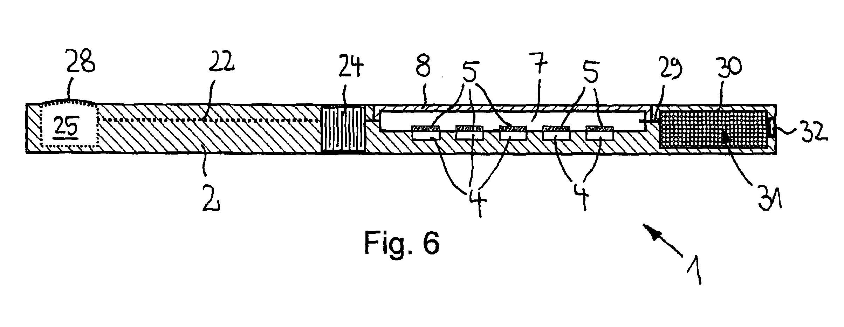

Bei dem In Fig. 6 gezeigten Ausführungsbeispiel weisen die Druckerzeugungskammem zum Verdrängen des darin enthaltenen Mediums In die Kapillaren 22, 23 Jeweils eine flexible Kammerwand 28 auf, die z.B. mit dem Finger oder einem geeigneten Werkzeug aus einer Ruhelage in Richtung auf das Medium zu auslenkbar ist.

Die Messkammer 7 hat eine zweite Öffnung 29, die mit einem Aufnahmebehälter 30 verbunden ist. In dem Aufnahmebehälter 30 ist ein saugfähiges Medium 31 angeordnet ist. Der Aufnahmebehälter 30 ist über ein Überdruckventil 32 mit der Atmosphäre verbunden.

**Patentansprüche**

1. Verfahren zur Bestimmung der Konzentration von in einer zu untersuchenden Probe enthaltenen Liganden (6), wobei an einer Teststelle auf einem Träger Rezeptoren (5) immobilisiert werden, die mit den Liganden (6) eine spezifische Bindung eingehen können, wobei die Probe mit der Teststelle in Kontakt gebracht wird, wobei mindestens ein die Häufigkeit der Bindungen zwischen den Liganden (6) und den Rezeptoren (5) repräsentierender erster Messwert erfasst wird, wobei danach eine Särtigungslösung, welche die Uganden (6) in höherer Konzentration enthält als die Probe, derart mit den Rezeptoren (5) in Kontakt gebraucht wird, dass im Wesentlichen alle an der Teststelle befindlichen Rezeptoren (5) an einen Liganden (6) gebunden sind, wobei danach ein die Häufigkeit der Bindungen zwischen den Liganden (6) und den Rezeptoren (5) repräsentierender zweiter Messwert erfasst wird, und wobei mit Hilfe des ersten und zweiten Messwerts die Konzentration der Uganden (6) in der Probe bestimmt wird.

2. Verfahren nach Anspruch 1, **dadurch gekennzeichnet, dass** die Probe mindestens zwei unterschiedliche Arten von Liganden (6) aufweist, dass jeder Ligandenart jeweils eine Rezeptorart zugeordnet ist, deren Rezeptoren (5) für die Liganden (6) der betreffenden Ligandenart bindungsspezifisch sind, dass auf dem Träger für jede dieser Rezeptorarten jeweils mindestens eine Teststelle eingerichtet wird, an der Rezeptoren (5) der betreffenden Rezeptorart immobilisiert werden, dass die Probe mit den Teststellen in Kontakt gebracht wird, dass für jede Teststelle mindestens ein die Häufigkeit der Bindungen zwischen den Liganden (6) und den Rezeptoren (5) der Teststelle repräsentierender erster Messwert erfasst wird, dass danach die Sättigungslösung, welche die Uganden (6) der einzelnen Ugandenarten jeweils in höherer Konzentration enthält als die Probe, derart mit den Teststellen in Kontakt gebraucht wird, dass im Wesentlichen alle an den Teststellen (5) befindlichen Rezeptoren an einen Liganden (6) gebunden sind, dass zur Bestimmung der Flächenbelegung der Teststellen mit den Liganden (6) für jede Rezeptorart Jeweils ein zweiter Messwert erfasst wird, und wobei mit Hilfe der ersten und zweiten Messwerte für jede Ligandenart jeweils die Konzentration der Liganden (6) in der Probe bestimmt wird.

3. Verfahren zur Bestimmung der Konzentration von in einer zu untersuchenden Probe enthaltenen Liganden (6),

   - wobei auf einem Träger an mindestens einer Teststelle Rezeptoren (5) immobilisiert werden, die mit den Liganden (6) eine spezifische Bindung eingehen können,
   - wobei die Probe während einer ersten Zeitdauer mit der mindestens einen Teststelle in Kontakt gebracht und danach von dieser entfernt wird,
   - wobei mindestens ein die Häufigkeit der Bindungen zwischen den Liganden (6) und den Rezeptoren (5) repräsentierender erster Messwert erfasst wird,
   - wobei die Probe für mindestens eine weitere Zeitdauer mit der Teststelle In Kontakt gebracht und danach von dieser entfernt wird,
   - wobei mindestens ein die Häufigkeit der Bindungen zwischen den Liganden (6) und den Rezeptoren (5) repräsentierender weiterer Messwert erfasst wird, und
   - wobei mit Hilfe des ersten und des mindestens einen weiteren Messwerts die Konzentration der Liganden (6) in der Probe bestimmt wird.

4. Verfahren nach Anspruch 3, zur Bestimmung der Konzentrationen von mindestens zwei in einer zu untersuchenden Probe enthaltenen Liganden (6) unterschiedlicher Ligandenarten, wobei Liganden (6) einer ersten Ugandenart eine höhere Konzentration aufweisen als Liganden (6) einer zweiten Ligandenart,

   - wobei auf einem Träger an einer ersten Teststelle Rezeptoren (5) einer ersten Rezeptorart und an einer zweiten Teststelle Rezeptoren (5) einer zweiten Rezeptorart immobilisiert werden,
   - wobei die Rezeptoren (5) der ersten Rezeptorart mit den Liganden (6) der ersten Ligandenart und die Rezeptoren (5) der zweiten Rezeptorart mit den Liganden (6) der zweiten Ligandenart jeweils eine spezifische Bindung eingehen können,
   - wobei die Probe während einer ersten Zeitdauer mit den Teststellen in Kontakt gebracht und danach von den Teststellen entfernt wird,
   - wobei mindestens ein die Häufigkeit der Bindungen zwischen den Liganden (6) der ersten Ugandenart und den Rezeptoren (5) der ersten Rezeptorart repräsentierender erster Messwert erfasst wird,
   - wobei die Probe für eine zweite Zeitdauer mit den Teststellen in Kontakt gebracht und danach von den Teststellen entfernt wird,
   - wobei mindestens ein die Häufigkeit der Bindungen zwischen den Uganden (6) der zweiten Ligandenart und den Rezeptoren (5) der zweiten Rezeptorart repräsentierender zweiter Messwert erfasst wird, und
   - wobei mit Hilfe der ersten und zweiten Messwerte die Konzentrationen der Liganden (6) in der Probe bestimmt werden.

**5.** Verfahren nach Anspruch 3 oder 4, **dadurch gekennzeichnet,**

- **dass** die Teststellen nach dem Erfassen des zweiten Messwerts mit einer Sättigungslösung, welche die Liganden (6) In höherer Konzentration enthält als die Probe, In Kontakt gebracht werden,
- **dass** danach ein die Häufigkeit der Bindungen zwischen den Liganden (6) der ersten Ligandenart und den Rezeptoren (5) der ersten Rezeptorart repräsentierender dritter Messwert sowie ein die Häufigkeit der Bindungen zwischen den Liganden (6) der zweiten Ugandenart und den Rezeptoren (5) der zweiten Rezeptorart repräsentierender vierter Messwert erfasst werden, und
- **dass** mit Hilfe der ersten, zweiten, dritten und vierten Messwerte die Konzentrationen der Liganden (6) in der Probe bestimmt werden.

**6.** Verfahren nach Anspruch 3 oder 4, **dadurch gekennzeichnet,**

- **dass** die erste Zeitdauer derart gewählt wird, dass nur ein Teil der an der ersten Teststelle befindlichen Rezeptoren (5) an einen Uganden (6) binden,
- **dass** die zweite Zeitdauer derart gewählt wird, dass an der ersten Teststelle Im Wesentlichen alle Rezeptoren (5) und an der zweiten Teststelle nur ein Teil der Rezeptoren (5) an einen Liganden (6) binden,
- **dass** nach Ablauf der zweiten Zeitdauer mindestens ein die Häufigkeit der Bindungen zwischen den Liganden (6) der ersten Ligandenart und den Rezeptoren (5) der ersten Rezeptorart repräsentierender dritter Messwert erfasst wird, und
- **dass** nach Ablauf der zweiten Zeitdauer mindestens eine Sättigungslösung, welche die Liganden (6) der zweiten Ligandenart in höherer Konzentration enthält als die Probe, derart zumindest mit den Rezeptoren (5) der zweiten Rezeptorart in Kontakt gebraucht wird, dass im Wesentlichen alle an der zweiten Teststelle befindlichen Rezeptoren (5) an einen Liganden (6) gebunden sind,
- **dass** danach ein die Häufigkeit der Bindungen zwischen den Liganden (6) der zweiten Ligandenart und den Rezeptoren (5) der zweiten Rezeptorart repräsentierender vierter Messwert erfasst werden, und
- **dass** mit Hilfe der ersten, zweiten, dritten und vierten Messwerte die Konzentrationen der Liganden (6) in der Probe bestimmt werden.

**7.** Verfahren nach einem der Ansprüche 1 bis 6, **da-**

**durch gekennzeichnet, dass** in Abhängigkeit von der Bindung der Liganden (6) an die Rezeptoren (5) Luminophoren und/oder Chemiluminophoren direkt und/oder indirekt über Nachweisantikörper (12) an die Liganden (6) gebunden werden, dass die Luminophoren und/oder Chemiluminophoren zur Abgabe von Lumineszenzstrahlung (21) angeregt werden, und dass die ersten und zweiten Messwerte durch Messung der Lumineszenzstrahlung (21) erfasst werden.

**8.** Verfahren nach einem der Ansprüche 1 bis 7, **dadurch gekennzeichnet, dass** auf dem Träger mindestens zwei Teststellen eingerichtet werden, auf denen Rezeptoren (5) unterschiedlicher Rezeptorarten mit unterschiedlicher Dichte immobilisiert werden.

**9.** Verfahren nach einem der Ansprüche 1 bis 8, **dadurch gekennzeichnet, dass** auf dem Träger mindestens zwei Teststellen eingerichtet werden, auf denen Rezeptoren (5) derselben Rezeptorart mit unterschiedlicher Dichte immobilisiertwerden.

**10.** Vorrichtung (1) zur Bestimmung der Konzentration von in einer zu untersuchenden Probe enthaltenen Liganden (6), mit einem Träger, an dessen Oberfläche mindestens eine Teststelle angeordnet ist, an der Rezeptoren (5) immobilisiert sind, die beim Kontaktieren der Liganden (6) mit diesen eine spezifische Bindung eingehen, mit wenigstens einem Detektor (4) zur Erfassung von Messwerten, die von der Häufigkeit der Liganden-Rezeptor-Bindungen abhängig sind, mit einem mit dem Detektor (4) verbundenen Datenspeicher zum Zwischenspeichern eines ersten, nach dem Inkontaktbringen der Probe mit der Teststelle erfassbaren Messwerts, mit einem Reservoir (9) für eine die Liganden (6) in hoher Konzentration enthaltende Sättigungslösung, mit einer Fördereinrichtung zum Fördern der Sättlgungslösung aus dem Reservoir (9) zu der mindestens einen Teststelle, und mit einer zumindest mit dem Datenspeicher und der Fördereinrichtung In Steuerverbindung stehenden Auswerte- und Steuereinrichtung (20), die derart ausgestaltet ist, dass nach dem Zwischenspeichern des ersten Messwerts die Sättigungslösung der Teststelle derart zugeführt wird, dass im Wesentlichen alle an der mindestens einen Teststelle befindlichen Rezeptoren (5) an einen Liganden (6) binden, und dass dann mit Hilfe des Detektors (4) ein zweiter Messwert für die Flächenbelegung der Teststelle mit den Liganden (6) erfasst und anhand des ersten und zweiten Messwerts ein Messsignal für die Konzentration der Liganden (6) In der Probe erzeugt wird.

**11.** Vorrichtung (1) nach Anspruch 10, **dadurch gekennzeichnet dass** zum Untersuchen einer mindestens zwei unterschiedliche Arten von Liganden (6) aufweisenden Probe mehrere Teststellen an der Oberfläche des Trägers vorgesehen sind, an denen Rezeptoren (5) immobilisiert sind, die für Liganden (6) unterschiedlicher Ligandenarten bindungsspezifisch sind, dass jeder Teststelle jeweils ein mit dem Datenspeicher verbundener Detektor (4) zugeordnet ist, dass der Datenspeicher zumindest eine der Anzahl der Teststellen entsprechende Anzahl Speicherplätze zum Zwischenspeichern von ersten, nach dem Inkontaktbringen der Probe mit der Teststelle erfassbaren Messwerten aufweist, und dass die Auswerte- und Steuereinrichtung (20) derart ausgestaltet ist, dass nach dem Zuführen der Sättigungslösung zu den Teststellen mit Hilfe der Detektoren (4) für jede Teststelle jeweils ein zweiter Messwert für die Flächenbelegung der Teststelle mit den Liganden (6) erfasst und anhand des ersten und zweiten Messwerts jeweils ein Messsignal für die Konzentration der für die Rezeptoren (5) der betreffende Teststelle bindungsspeziflschen Liganden (6) in der Probe erzeugt wird.

12. Vorrichtung (1) zur Bestimmung der Konzentrationen von in einer zu untersuchenden Probe enthaltenen Liganden (6) zumindest einer ersten und einer zweiten Ligandenart, mit einem Träger, an dessen Oberfläche zumindest eine erste und eine zweite zwei Teststelle angeordnet sind, wobei an der ersten Teststelle Rezeptoren (5) einer ersten Rezeptorart und an einer zweiten Teststelle Rezeptoren (5) einer zweiten Rezeptorart immobilisiert sind, wobei die Rezeptoren (5) der ersten Rezeptorart mit den Liganden (6) der ersten Ligandenart und die Rezeptoren (5) der zweiten Rezeptorart mit den Liganden (6) der zweiten Ligandenart jeweils eine spezifische Bindung eingehen können, mit wenigstens einem Detektor (4) zur Erfassung von Messwerten, die von der Häufigkeit der Liganden-Rezeptor-Bindungen abhängig sind, mit einem mit dem Detektor (4) verbundenen Datenspeicher zum Zwischenspelchem zumindest eines ersten, nach dem Inkontaktbringen der Probe mit der Teststelle erfassbaren Messwerts für jede Detektorart, und mit einer mit dem Reservoir (9) und der Teststelle verbundenen Fördereinrichtung, mit dem die Probe von dem Reservoir (9) zu der Teststelle und von der Teststelle zu dem Reservoir förderbar ist.

13. Vorrichtung (1) nach einem der Ansprüche 10 bis 12, **dadurch gekennzeichnet, dass** sie zusätzlich zu dem ersten Reservoir (9) mindestens ein zweites Reservoir (10) aufweist, in dem ein mit einem optischen Marker (11) markierter oder markierbarer Nachweisantikörper (12) angeordnet ist, der dazu geeignet ist, an die Liganden (6) zu binden, dass der Marker (11) mittels der Fördereinrichtung der mindestens einen Teststelle zuführbar ist, und dass der Detektor (4) als optischer Detektor zum Erfassen einer bei Anwesenheit des Markers (11) auftretenden Lumineszenzstrahlung (21) ausgebildet ist.

14. Vorrichtung (1) nach einem der Ansprüche 10 bis 13, **dadurch gekennzeichnet, dass** sie ein drittes Reservoir (13) aufweist, in dem ein Cheml-Luminophor angeordnet ist, der bei einem Kontakt mit dem Marker (11) zur Abgabe einer Chemi-Lumineszenzstrahlung anregbar ist, und dass der Chemi-Luminophor mittels der Fördereinrichtung der mindestens einen Teststelle zuführbar ist.

15. Vorrichtung (1) nach einem der Ansprüche 10 bis 14, **dadurch gekennzeichnet, dass** sie mindestens ein viertes Reservoir (14) aufweist, in dem eine Spülflüssigkeit angeordnet ist, und dass die Spülflüssigkeit mittels der Fördereinrichtung der mindestens einen Teststelle zuführbar ist.

16. Vorrichtung (1) nach einem der Ansprüche 10 bis 15, **dadurch gekennzeichnet, dass** sie ein fünftes Reservoir aufweist, in dem Wasserstoffperoxid angeordnet ist, und dass das Wasserstoffperoxid mittels der Fördereinrichtung der mindestens einen Teststelle zuführbar ist.

17. Vorrichtung (1) nach einem der Ansprüche 10 bis 16, **dadurch gekennzeichnet, dass** das erste Reservoir (9) und das zweite Reservoir (10) sowie ggf das dritte Reservoir (12) und/oder das vierte Reservoir (14) durch mit Flüssigkeiten befüllte, hintereinander angeordnete Abschnitte einer Kapillare (22, 23) gebildet sind, die an einem Abgabeende mit einer die mindestens eine Teststelle aufweisenden Messkammer (7) verbunden ist, und dass die In den Reservoiren (10) gespeicherten Flüssigkeiten mittels der Fördereinrichtung entlang der Kapillare (22, 23) in die Messkammer (7) verschiebbar sind.

18. Vorrichtung (1) nach einem der Ansprüche 10 bis 17, **dadurch gekennzeichnet, dass** zwischen zueinander benachbarten Reservoiren (9, 10, 13, 14) in der Kapillare (22, 23) Jeweils eine Diffusionssperre angeordnet ist, die vorzugsweise durch eine hydrophobe Flüssigkeit gebildet Ist.

19. Vorrichtung (1) nach einem der Ansprüche 10 bis 18, **dadurch gekennzeichnet, dass** sie zusätzlich zu der ersten Kapillare (22) mindestens eine zweite Kapillare (23) aufweist, die mit dem Wasserstoffperoxid befüllt ist, und dass die erste Kapillare (22) und die zweite Kapillare (23) jeweils mit einer Einlassöffnung eines Mischers (24) verbunden sind, der mit einer Auslassöffnung an der Messkammer (7) angeschlossen ist.

20. Vorrichtung (1) nach einem der Ansprüche 10 bis 19, **dadurch gekennzeichnet, dass** die Fördereinrichtung mindestens eine mit einem fließfähigen Medium befüllte Druckerzeugungskammer (25) aufweist, die zum Verdrängen der in der Kapillare (22, 23) bevorrateten Flüssigkeiten mit dem von der Messkammer (7) entfernten Ende der Kapillare (22, 23) verbunden ist, und dass die Druckerzeugungskammer (25) eine Einrichtung zum Erzeugen eines hydraulischen und/oder pneumatischen Drucks in dem fließfähigen Medium aufweist.

21. Vorrichtung (1) nach einem der Ansprüche 10 bis 20, **dadurch gekennzeichnet, dass** die Einrichtung zum Erzeugen des hydraulischen und/oder pneumatischen Drucks eine Heizeinrichtung (26, 27) zum Beheizen des in der Druckerzeugungskammer (25) befindlichen fließfähigen Mediums aufweist.

22. Vorrichtung (1) nach einem der Ansprüche 10 bis 21, **dadurch gekennzeichnet, dass** die Druckerzeugungskammer (25) eine verformbare Kammerwand aufweist, die durch Druckbeaufschlagung an ihrer Außenseite in eine Innenhöhlung der Druckerzeugungskammer (25) auslenkbar ist.

23. Vorrichtung (1) nach einem der Ansprüche 10 bis 22, **dadurch gekennzeichnet, dass** die Messkammer (7) eine mit einem Aufnahmebehälter (30) verbundene Auslassöffnung (29) hat, und dass in dem Aufnahmebehälter (30) ein saugfähiges Medium (31) angeordnet ist.

Fig. 1a

Fig. 1b

Fig. 1c

$H_2O_2$

11

HRP

$H_2O + O$

+ Luminol

12

Luminol ox. + h*n

6

21

7

428 nm

5

4

3

Fig. 2

Fig. 3

Fig. 4

EP 1 801 588 A1

Fig. 5

Fig. 6

# EP 1 801 588 A1

**Europäisches Patentamt**

**Nummer der Anmeldung**

EP 05 02 8312

## EUROPÄISCHER RECHERCHENBERICHT

### EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | US 2002/045277 A1 (SCHMID BEATE ET AL) 18. April 2002 (2002-04-18) * Absatz [0016] * * Absätze [0031] - [0035] * ----- | 1,2 | INV. G01N33/543 |
| X | MONTREL M M ET AL: "Spectroscopic study of thin multilayer films of the complexes of nucleic acids with cationic amphiphiles and polycations: Their possible use as sensor elements" SENSORS AND ACTUATORS B, ELSEVIER SEQUOIA S.A., LAUSANNE, CH, Bd. 42, Nr. 3, August 1997 (1997-08), Seiten 225-231, XP004100268 ISSN: 0925-4005 * Seite 227 - Seite 229 * * Abbildung 4 * ----- | 3-9 | |
| X | DE 197 36 641 A1 (WELLER, MICHAEL G., DR., 81375 MUENCHEN, DE; NIESNER, REINHARD, PROF.) 11. März 1999 (1999-03-11) * Abbildungen 1,2 * ----- | 10-23 | |
| X | WO 2004/042399 A (MICRONAS HOLDING GMBH; KLAPPROTH, HOLGER) 21. Mai 2004 (2004-05-21) * Seite 7 - Seite 10 * ----- | 10-23 | RECHERCHIERTE SACHGEBIETE (IPC) G01N |
| X | US 5 324 633 A (FODOR ET AL) 28. Juni 1994 (1994-06-28) * Spalte 6, Zeile 62 - Spalte 7, Zeile 9 * * Spalte 12 * ----- | 10-23 | |

Der vorliegende Recherchenbericht wurde für alle Patentansprüche erstellt

| Recherchenort | Abschlußdatum der Recherche | Prüfer |
|---|---|---|
| Den Haag | 29. Mai 2006 | Lunter, P |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

22

## ANHANG ZUM EUROPÄISCHEN RECHERCHENBERICHT
## ÜBER DIE EUROPÄISCHE PATENTANMELDUNG NR.

EP 05 02 8312

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

29-05-2006

| Im Recherchenbericht angeführtes Patentdokument | Datum der Veröffentlichung | Mitglied(er) der Patentfamilie | | Datum der Veröffentlichung |
|---|---|---|---|---|
| US 2002045277 A1 | 18-04-2002 | CA<br>DE<br>EP | 2358652 A1<br>10050632 A1<br>1209470 A2 | 12-04-2002<br>18-04-2002<br>29-05-2002 |
| DE 19736641 A1 | 11-03-1999 | KEINE | | |
| WO 2004042399 A | 21-05-2004 | DE<br>EP<br>JP<br>US | 10251757 A1<br>1451586 A1<br>2006505772 T<br>2005239132 A1 | 19-05-2004<br>01-09-2004<br>16-02-2006<br>27-10-2005 |
| US 5324633 A | 28-06-1994 | KEINE | | |

EPO FORM P0461

Für nähere Einzelheiten zu diesem Anhang : siehe Amtsblatt des Europäischen Patentamts, Nr.12/82

**EP 1 801 588 A1**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2004042399 A1